# EUROPÄISCHE PATENTSCHRIFT

(11) **EP 2 370 129 B1**
(45) Veröffentlichungstag und Bekanntmachung des Hinweises auf die Patenterteilung: **28.01.2015**
(21) Anmeldenummer: 09804185.8
(22) Anmeldetag: 19.11.2009
(51) Int. Cl.: A61M 5/145

(54) **ANÄSTHESIESPRITZE MIT EINEM LÄNGSVERSCHIEBLICHEN VORSCHUBKOLBEN SOWIE RÜCKSCHLAGVENTIL MIT DURCHLASS UND SPERRRICHTUNG**
ANESTHETIC SYRINGE HAVING A LONGITUDINALLY DISPLACEABLE FEEDING PISTON AND CHECK VALVE HAVING PASSAGE AND BLOCKING DEVICE
SERINGUE POUR ANESTHÉSIE MUNIE D'UN PISTON D'AVANCEMENT COULISSANT LONGITUDINALEMENT AINSI QU'UN CLAPET DE RETENUE AVEC PASSAGE ET SENS D'ARRÊT

(30) Priorität: 19.11.2008 DE 102008058213
(43) Veröffentlichungstag der Anmeldung: 05.10.2011
(73) Patentinhaber: SMJM Inject GmbH, 52062 Aachen (DE)
(72) Erfinder: STAMMEN, Christian, 41564 Kaarst-Driesch (DE); WAGNER, Georg, 56651 Niederzissen (DE); REINERTZ, Olivier, Georg, 4700 Eupen (BE); JANSEN, Roman, Josef, 41849 Wassenberg (DE)
(74) Vertreter: Castell, Klaus
(86) Internationale Anmeldenummer: PCT/DE2009/001634
(87) Internationale Veröffentlichungsnummer: WO 2010/057473

(56) Entgegenhaltungen:
- WO-A1-02/49697
- WO-A1-2005/075009
- WO-A2-02/081009
- DE-A1- 19 614 337

## Beschreibung

Die Erfindung betrifft eine Spritze, insbesondere eine Anästhesiespritze mit einem längsverschieblichen Vorschubkolben, welcher über eine erste Hydraulikkammer verschiebbar angeordnet ist und einer zweiten Hydraulikkammer, wobei die erste und zweite Hydraulikkammer widerstandsregulierbar über ein erstes Steuerelement verbunden sind, wobei das erste Steuerelement über ein Schaltelement betätigbar ist und das Schaltelement ein Betätigungsstift ist..

In vielen Bereichen der Medizin stellt sich regelmäßig die Aufgabe, Anästhesiemittel in einen menschlichen oder tierischen Körper zu injizieren. Hierzu werden Anästhesiespritzen verwendet, die sich auch im Wesentlichen bewährt haben.

Der weit überwiegende Teil der in der Praxis verwendeten Anästhesiespritzen ist manuell zu bedienen. Diese Art der Anästhesiespritzen bedarf aufgrund Ihrer allgemeinen Bekanntheit keiner Erläuterung.

In der WO 2005/075009 A1 wird eine sehr handliche Anästhesiespritze zur Verfügung gestellt, wodurch Injektionsvorgänge präzise und wiederholbar durchführbar sind. Diese Anästhesiespritze weist eine erste und eine zweite Hydraulikkammer auf, welche über einen der Vertikalschieber widerstandsregulierbar oder entsprechend steuerbar verbunden sind. Um nach einem Einspritzvorgang die Anästhesiespritze wieder in die Ausgangsposition zu bringen, muss dieser Vertikalschieber manuell oder entsprechend mit Kraftaufwand in seine Ursprungsposition gebracht werden.

Eine gattungsgemäße Spritze ist aus der WO 02/49697 A1 bekannt.

Aufgabe der Erfindung ist es, über nur eine Verbindung der Hydraulikkammerneinen bidirektionalen Flüssigkeitsaustausch zu ermöglichen. Durch Einsparung von Bauteilen soll die Spritze kompakter gestaltet werden.

Gelöst wird diese Aufgabe durch eine Spritze, mit den Merkmalen des Patentanspruchs 1. Über den Vorschubkolben kann ein Medikament in einem Karpulenraum über eine angebrachte Injektionsnadel in ein Gewebe eingebracht werden.

Dadurch kann vorteilhafterweise die Rückstellung der Spritze ohne Betätigung eines Auslösehebels erfolgen, da das Rückschlagventil selbständig öffnet und die Hydraulikflüssigkeit somit von der ersten Hydraulikkammer in die zweite Hydraulikkammer befördert werden kann.

Rückschlagventile der hier genannten Art können insbesondere Standardrückschlagventile umfassen, welche als Fertigbauteile erwerbbar sind. Dadurch kann vorteilhafterweise der Herstellungspreis für eine solche Spritze reduziert werden.

In einer Ausprägungsform der Erfindung kann zwischen erster und zweiter Hydraulikkammer ein Widerstandselement in Reihe zum ersten Rückschlagventil geschaltet sein und somit insbesondere das erste Steuerelement, ein erstes Rückschlagventil und ein Widerstandselement umfassen. Dadurch kann vorteilhafterweise ein Widerstand eingestellt werden, wodurch die Injektion homogener und für den Patienten angenehmer verläuft.

Das Widerstandselement kann als Drossel oder Blende ausgestaltet sein.

Um den Vorschubkolben bei enthaltenem Widerstandselement nach Abschnitt 9 möglichst mit wenig Kraftaufwand wieder in die ursprüngliche Position nach einem Injektionsvorgang zu bringen, kann zwischen erster und zweiter Hydraulikkammer ein zweites Steuerelement, insbesondere ein zweites Rückschlagventil parallel zum ersten Steuerelement geschaltet sein. Dadurch kann, bei manueller Betätigung des Vorschubkolbens, die Hydraulikflüssigkeit aus der ersten Hydraulikkammer über das zweite Steuerelement in die zweite Hydraulikkammer fließen, ohne dass dabei der erhöhte Durchflusswiderstand des ersten Steuerelements spürbar wird.

In einer Ausgestaltungsform der Erfindung kann das Schaltelement als Pin oder entsprechend als ein Betätigungsstift ausgestaltet sein, welcher verschiebbar ist und bei Betätigung das erste Steuerelement öffnet, so dass die erste und zweite Hydraulikkammer mit einander leitend verbunden sind. Somit kann vorteilhafterweise eine einfache Betätigung des Steuerelements erfolgen.

Damit die, die Spritze betätigende Person ein Gefühl über den Druck in der Karpule erhält, kann das Schaltelement eine Wirkfläche aufweisen, welche wenigstens einen Teil des in der Spritze wirkenden hydraulischen Drucks und somit einer wirkenden Kraft mittels des Stellelements an den Benutzer überträgt.

In einer weiteren Ausprägungsform der Erfindung kann die wirkende Kraft verstärkt werden. Dies kann insbesondere dann vorteilhaft sein, wenn zum Betätigen des Steuerelements eine Kraft ausreicht, welche die betätigende Person kaum fühlt, jedoch die dann wirkende Kraft über die Wirkfläche haptisch erfasst. Um diese Kraft über ein Schaltsystem an dem Benutzer rückzukoppeln, kann vorteilhafterweise die Verstärkung der wirkenden Kraft über einen weiteren Pin mit einer Kraftwirkung über einen Hebel realisiert werden.

In einer weiteren Ausprägungsform der Erfindung kann die Verstärkung der Kraft über ein Hebelsystem, von zwei miteinander verschaltenden Hebeln erfolgen. Dabei ist der Hebelarm, welcher den Pin für das Stellelement betätigt nahe am Hebelpunkt oder entsprechend Hebelachse des zweiten Hebels verortet. Idealerweise wird der zweite Hebel am Ende des zweiten Hebelarms betätigt.

Um ein Widerstandselement zu realisieren kann das Widerstandselement als Spalt mit einer Spaltlänge um das Schaltelement, insbesondere um den Pin, ausgestaltet sein. Dabei umfließt die Hydraulikflüssigkeit den Pin entlang des Spaltes, welcher als Widerstand für die Hydraulikflüssigkeit, welche von der zweiten Hydraulikkammer in die erste Hydraulikkammer fließt, wirkt.

In einer weiteren Ausprägungsform der Erfindung kann die Spaltlänge so ausgestaltet sein, dass sich die Spaltlänge beim Betätigen des Schaltelements verändert. Dadurch kann vorteilhafterweise ein nichtkonstanter Widerstand realisiert werden.

Um einen Gasraum zu befüllen, welcher insbesondere zur Bereitstellung der Energie in der Spritze dient, kann die Spritze einen Gasraum umfassen, welcher über einen beweglichen Trennkolben oder eine Membrane mit der zweiten Hydraulikkammer verbunden ist, wobei dieser Gasraum über einen Befüllstutzen befüllbar ist, wobei der Gasraum über einen O-Ring gegenüber dem Befüllstutzen abgedichtet ist, wobei der O-Ring so ausgestaltet ist, dass die Funktion eines Rückschlagventils ausgeprägt wird. Durch diese Ausgestaltungsform kann vorteilhafterweise eine einfache Befüllung des Gasraums realisiert werden, wobei nach der Befüllung der Gasraum im Wesentlichen hermetisch abgedichtet ist.

Sowohl der hier aufgeführte O-Ring als auch sämtliche weiteren in diesem Dokument aufgeführten O-Ringe können Dichtungen, welche insbesondere als Ringe ausgestaltet sind, vorspannen und somit ein Gleitdichtsystem aus O-Ring und gespannter Dichtung bilden.

Um ein Begasen der Hydraulikflüssigkeit in der zweiten Hydraulikkammer zu verhindern, kann der Trennkolben zwei Dichtelemente, welche je den Gasraum und die zweite Hydraulikkammer abdichten, einen Kolbensystem ausbilden, wobei das Kolbensystem entlang eines Begrenzungselements führbar ist, wobei die Dichtungselemente, der Trennkolben und das Begrenzungssystem einen Hohlraum ausbilden, welcher eine Öffnung zur Umgebung aufweist.

Vorteilhafterweise ist die Spritze so ausgestaltet, dass die Öffnung in allen Trennkolbenpositionen austauschenden Kontakt zwischen Hohlraum und Umgebung ausbildet. Dadurch kann vorteilhafterweise das Begasen der Hydraulikflüssigkeit über alle Kolbenpositionen verhindert werden.

In einer weiteren Ausprägungsform der Erfindung kann die zweite Hydraulikkammer vollständig durch einen eigenständigen in sich vollständig geschlossenen Gasspeicher, der mit der zweiten Hydraulikkammer verbunden ist, separiert werden, wodurch vorteilhafterweise ein begasen der zweiten Hydraulikkammer verhindert werden kann.

Der eigenständige, in sich vollständig geschlossenen Gasspeicher kann als faltbares Kolbenelement ausgeführt sein, wobei das Kolbenelement entlang der Hauptbewegungsachse der Spritze stark deformierbar und radial kaum deformierbar ist, wobei das Kolbenelement über einen Befüllstutzen befüllbar ist, wobei der Gasraum im inneren des Kolbenelementes über einen O-Ring gegenüber dem Befüllstutzen abgedichtet ist, wobei der O-Ring so ausgestaltet ist, dass die Funktion eines Rückschlagventils ausgeprägt wird. Durch diese Ausgestaltungsform kann vorteilhafterweise eine einfache Befüllung des Gasraums realisiert werden, wobei nach der Befüllung der Gasraum im Wesentlichen hermetisch abgedichtet ist.

Dieser O-Ring kann insbesondere so weitergebildet werden, dass mit diesem ein Gleitdichtsystem gebildet wird, welches an die Position des O-Rings tritt.

Faltbare Kolbenelemente der hier genannten Art können insbesondere Federmembranspeicher umfassen, welche als Fertigbauteile erwerbbar sind.

Zur bedarfsweisen Entlüftung des vollständig geschlossenen Gasspeichers kann eine verschließbare Entlüftungsöffnung integriert sein, wobei die Entlüftung dergestallt ausgeführt ist, dass die Entlüftung des Gastraums vorteilshafterweise vor der Trennung des Gasspeichers von der zweiten Hydraulikkammer erfolgt. Durch diese Ausgestaltungsform kann vorteilshafterweise eine gefahrlose Trennung des Gasspeichers von der zweiten Hydraulikkammer realisiert werden.

In einem weiteren Aspekt der Erfindung kann ein Rückschlagventil realisiert werden, wobei das Rückschlagventil eine Durchlass- und Sperrrichtung ausbildet, wobei das Rückschlagventil einen ersten mit einem zweiten Raum verbindet, wobei das Rückschlagventil mittels O-Ring ausgeführt ist, welcher eine Nut umschließt. Dabei weist die Nut eine Verbindung zum ersten Raum auf und der O-Ring ist im Wesentlichem im zweiten Raum verortet. Somit dichtet der O-Ring den zweiten vom ersten Raum ab, sofern der zweite Raum einen höheren Druck als der erste Raum aufweist. Für den Fall, dass der erste Raum einen höheren Druck als der zweite Raum aufweist, dehnt sich der O-Ring aus und eine leitende Verbindung vom ersten zum zweiten Raum wird ausgebildet. Dadurch kann ein Medium vom ersten in den zweiten Raum geleitet werden.

Dieser hier aufgeführte O-Ring kann ebenfalls insbesondere so weitergebildet werden, dass mit diesem ein Gleitdichtsystem gebildet wird, welches an die Position des O-Rings tritt.

O-Ringe und Gleitdichtsysteme der hier genannten Art können sämtliche Dichtelemente umfassen, welche aufgrund ihrer Elastizität einen Raum von einem anderen abdichten, wobei die gespannten Ringe der Gleitdichtsysteme PTFE, KFM und FFKM umfassen können. Insbesondere die Materialien KFM (Fluorkautschuk) und FFKM können in Systemen eingesetzt werden, in denen die vorgespannten Ringe einer aggressiven chemischen Umgebung ausgesetzt sind. Insbesondere können die vorgespannten Ringe der Firma Trellborg eingesetzt werden, die unter den registrierten Markennamen, Turcon, Stepseal 2K und/oder Zurcon geführt werden.

In einer Ausprägungsform der Erfindung kann der O-Ring in Durchlassrichtung radial nach Außen dehnbar ausgestaltet sein und in Sperrrichtung die Funktion des Abdichtens erfüllen. Dadurch können vorteilhafterweise Standard- O-Ringe wie sie im Handel erhältlich sind, eingesetzt werden.

In einem weiteren Aspekt der Erfindung wird ein O-Ring als Rückschlagventil verwendet.

Im weiterem wird die Erfindung anhand von Ausführungsbeispielen erläutert.

Dabei zeigt
- Figur 1a: eine Anästhesiespritze mit einer seriellen Anordnung aus Karpulenraum, Vorschubkolben, erster Hydraulikkammer, zweiter Hydraulikkammer, Trennkolben und Druckraum,
- Figur 1b: eine Anästhesiespritze mit einer seriellen Anordnung aus Karpulenraum, Vorschubkolben, erster Hydraulikkammer, zweiter Hydraulikkammer und Gasraum, wobei im Gasraum ein Membranbalg angeordnet ist,
- Figur 1c: ein Gleitsystem, bei dem ein O-Ring einen PTFE-Ring vorspannt.
- Figur 2: eine erfindungsgemäße Spritze mit erstem ond zweiten Hydraulikraum, welche über ein Rückschlagventil verbunden sind,
- Figur 3: eine erfindungsgemäße Spritze mit ersten und zweiten Hydraulikraum, welche über ein Rückschlagventil verbunden sind, wobei zwischen Rückschlagventil und zweitem Hydraulikraum ein Widerstandselement angeordnet ist,
- Figur 4: die Spritze aus Figur 3, welche um ein zweites Steuerelement erweitert ist,
- Figur 5: einen Pin mit Wirkfläche, welcher das Steuerelement über einen Hebel beschaltet,
- Figur 6: ein System bei dem die wirkenden Kräfte verstärkt werden,
- Figur 7: ein Hebelsystem von zwei miteinander verschaltenden Hebeln,
- Figur 8: ein Schaltelement mit einem Spalt und einer Spaltlänge über den gesamten Steuerbereich,
- Figur 9: eine Spaltlänge, welche sich beim Betätigen des Schaltelements verändert,
- Figur 10: einen O-Ring als Rückschlagventil und das dazugehörge Ersatzschaltbild,
- Figur 11: eine Entgasungsvorrichtung in der Spritze.

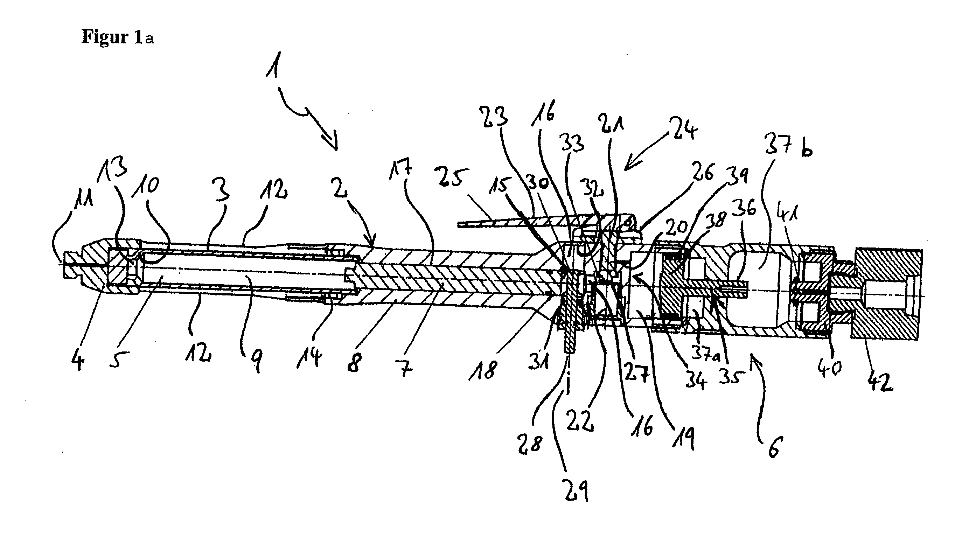

Die Anästhesiespritze 1 in der Figur 1a besteht im Wesentlichen aus einem Gehäuse 2, welches in einem Karpulenraum 3 einer Karpulenhülse 4 eine Karpule 5 aufnehmen und deren Inhalt mit einem hydraulisch-pneumatischen Antriebssystem 6 über einen Vorschubkolben 7 austreiben kann.

Der Vorschubkolben 7 ist in einem Vorschubzylinder 8 entlang einer Haupterstreckungsachse 9 der Anästhesiespritze 1 verschiebbar gelagert, wobei der Vorschubkolben 7 in der Figur eine innere Endlage einnimmt und bei einem Vorschub entlang der Achse 9 bis zu einer Anschlagschulter 10 bewegt werden kann, wo er eine äußere Endlage einnimmt. Beim Vorschub durchfährt der Vorschubkolben 7 den Karpulenraum 3, so dass Flüssigkeit aus einer eingelegten Karpule 5 durch eine Nadelöffnung 11 ausgespritzt werden kann. Zum Einsatz der Spritze 1 wird in die Nadelöffnung 11 eine Kanüle eingesetzt. Die Kanüle hat eine Spitze zum Einstechen ins Gewebe und eine zweite Spitze zum Einstechen in die Dichtmembran der eingelegten Karpule 5.

In der Nadelöffnung 11 ist eine Spezialaufnahme für die Kanüle vorgesehen, (nicht im Detail dargestellt), damit nicht versehentlich eine Kanüle in die Spritze 1 eingesetzt werden kann, welche bei den mitunter hohen Drücken mechanisch versagt.

Die Karpulenhülse 4 hat zwei Sichtfenster 12, durch welche die Vorschubposition des Vorschubkolbens 7 innerhalb der Karpulenhülse 4 einsehbar ist. Die Karpule 5 hat eine handelsübliche Kopfgestaltung 13, so dass der behandelnde Arzt nicht in der Wahl der Karpulen eingeschränkt wird, welche er bereits von der Arbeit mit herkömmlichen Spritzen kannte. Die Karpulenhülse 4 ist über einen Bajonettverschluss 14 mit dem Vorschubzylinder 8 fest verbunden.

Mit einer Druckplatte 15 ist der Vorschubkolben 7 einer ersten Hydraulikkammer 16 zugewandt, wobei eine Mantelfläche 17 des Vorschubkolbens 7 über einen O-Ring 18 gegenüber der ersten Hydraulikkammer 16 abgedichtet ist.

Rückwärtig der ersten Hydraulikkammer 16 ist eine zweite Hydraulikkammer 19 vorgesehen und mit der ersten Hydraulikkammer 16 über eine Steuerbohrung 20 verbunden. Die Öffnung der Steuerbohrung 20 zwischen der ersten Hydraulikkammer 16 und der zweiten Hydraulikkammer 19 regelt ein Ventilschieber 21, welcher entlang einer Bewegungsrichtung 22 verschiebbar gelagert ist und an einer Außenseite des Gehäuses 2 an einen Tasterarm 23 eines Steuertasters 24 stößt. Dem vorderen Bereich der Spritze 1 zugewandt weist der Steuertaster 24 eine Tastfläche 25 zum Niederdrücken des Tasterarms 23 - und somit des Ventilschiebers 21 - auf. Hierzu ist der Tasterarm 23 an einem Tasterlager 26 um einen Tasterbolzen drehbar gelagert. Der Tasterbolzen liegt senkrecht zur Haupterstreckungsachse 9 der Anästhesiespritze 1.

Der Ventilschieber 21 ragt mit einer Druckplatte 27 in die erste Hydraulikkammer hinein, während die Mantelfläche des Ventilschiebers 21 mit einem O-Ring abgedichtet ist.

In ähnlicher Weise ist ein Indexkolben 28 im Gehäuse 2. entlang einer zur Haupterstreckungsrichtung 9 der Spritze 1 radialen Richtung 29 gelagert. Der Indexkolben 28 ragt mit einem Fuß 30 in die erste Hydraulikkammer 16 hinein, während die Mantelfläche des Indexkolbens 28 mit einem O-Ring 31 abgedichtet ist. Eine Druckfeder (nicht dargestellt) presst den Indexkolben 28 zu einer inneren Endlage, bei welcher der Fuß 30 an einer inneren Begrenzungsfläche 32 zum Liegen kommt (in der Figur ist der Indexkolben 28 zur besseren Übersichtlichkeit bis zu einem Anschlag ausgefahren dargestellt; ohne Druck in der Hydraulikkammer 1 nimmt der Indexkolben bei der vorliegenden Ausführung 1 jedoch in der Praxis die innere Endposition ein).

Eine weitere Druckfeder 33 presst den Ventilschieber 21 unter Vorspannung im Ruhezustand in die dargestellte äußere Endlage, welche durch Anschlag der gegenüber dem zylindrischen Teil des Schiebers 21 vergrößert ausgeführten Druckplatte 27 an das Gehäuse 2 an einem Anschlag 34 definiert wird.

Die zweite Hydraulikkammer 19 ist rückwärtig von einem Trennkolben 35 begrenzt, welcher entlang der Haupterstreckungsrichtung 9 der Spritze 1 verschiebbar gelagert ist und seinerseits rückwärtig in einen Druckraum 37a, 37b ragt und diesen somit begrenzt. Eine Druckplatte 38 bildet das wesentliche Element des Trennkolbens 35. An Seiten der Druckplatte 38 ist eine Doppel-O-Ring-Dichtung 39 vorgesehen. Damit der Trennkolben 35 bei einer Bewegung zwischen der zweiten Hydraulikkammer 19 und dem Druckraum 37a nicht verkantet, ist im Druckraum 37, 37b eine Führung für einen zylindrischen Führungsstab 36 des Trennkolbens 35 vorgesehen. Große Durchlässe verbinden die beiden Teilkammern 37a und 37b des Druckraums (in der Figur nicht dargestellt).

Der Druckraum 37b wird rückwärtig durch einen Verschlussstopfen 40 begrenzt, welcher über einen weiteren O-Ring 41 dicht an den Druckraum 37 angeschlossen ist und mit einem Rückschlagventil (nicht im Detail dargestellt) versehen ist, über welches eine Gaspatrone oder ein Adapter 42 zum Anschluss einer Druckgasleitung mit der Spritze 1 verbunden ist.

Im Betrieb wird der notwendige Hydraulikdruck zum Vorschub des Vorschubkolbens 7 vom Druckraum 37 erzeugt. Der Trennkolben 35 trennt dabei das Gasvolumen (unter Druck über den Adapter 42 in den Druckraum 37 gefüllt) vom Hydrauliköl in der zweiten Hydraulikkammer 19. Über die Steuerbohrung 20 kann das unter Druck stehende Hydrauliköl zum Ventilschieber 21 gelangen. Der Ventilschieber wird über die Druckfeder 33 im Ruhezustand verschlossen. Die Druckfeder ist über einen Gewindedeckel (nicht beziffert, im Gehäuse 2 dem Ventilschieber 21 auf der gegenüberliegenden Seite in dessen Verlängerung angeordnet) vorgespannt, um die notwendige Dichtkraft an der Steuerbohrung 20 zu erzeugen. Das Ventil ist ein 2/2-Wege-Sitzventil mit proportionaler Charakteristik, somit kann abhängig vom Ventilhub der Volumenstrom und damit die Ausfuhrgeschwindigkeit des Vorschubkolbens 7 gesteuert werden.

Um den Vorschubkolben 7 auszufahren, muss der Tasterarm 23 bevorzugt an dessen Tastfläche 25 zum Gehäuse 2 der Spritze 1 hin betätigt werden. Dies verschiebt zwangsweise den Ventilschieber 21 entlang seiner Bewegungsrichtung 22 gegen die Druckfeder 33 in das Gehäuse 2 der Spritze 1 hinein. Der freigegebene Strömungsquerschnitt kann nun vom Hydrauliköl von der zweiten Hydraulikkammer 19 zur ersten Hydraulikkammer 16 hin durchflossen werden. Somit wird der Kolbenboden 15 des Vorschubkolbens 7 mit Druck beaufschlagt, so dass der Vorschubkolben 7 ausfährt.

Liegt über das Gewebe, in welches die Spritze 1 eingestochen ist, eine Gegenkraft am Vorschubkolben 7 an, so steigt der Druck in beiden Hydraulikkammern, auf Grund der hydraulischen Verluste an der Steuerbohrung 20 jedoch insbesondere in der ersten Hydraulikkammer 16. Dieser Druck wirkt auf den Fuß 27 des Ventilschiebers 21 in Schließrichtung des Ventils. Der behandelnde Arzt spürt über den Taster 24 den veränderten Druck und muss entsprechend stärker auf den Taster 24 drücken, damit der Ventilschieber 21 in der geöffneten Position und damit das Ventil der Steuerbohrung 20 geöffnet bleibt.

Zusätzlich zu dieser Drucksensierung ist eine optische Druckanzeige über den Indexkolben 28 vorhanden, die den Zylinderdruck und damit die Kolbenkraft - welche als Maß für den Injektionsdruck angesehen werden kann - anzeigt. Dabei fungiert der Indexkolben 28 als kleiner einfachwirkender Hydraulikzylinder, an dessen Kolben drei Nuten zur Anzeige der Druckstärke eingebracht sind. Der Kolben 28 wird von der Feder im Ruhezustand im Gehäuse 2 gehalten (dieser Zustand ist hier nicht dargestellt). Bei ansteigendem Hydraulikdruck in der ersten Hydraulikkammer entsteht über den Fuß 30 des Indexkolbens 28 eine Kraft, welche der Federvorspannung entgegenwirkt und den Indexkolben 28 entsprechend der hydraulischen Druckverhältnisse in der ersten Hydraulikkammer 16 entlang der Bewegungsrichtung 29 aus dem Gehäuse 2 der Spritze 1 mehr oder weniger weit ausfährt. Über die Nuten am Kolben 28 kann dann der Druck abgelesen werden.

Ist der Vorschubkolben 7 komplett ausgefahren und somit die Glaskarpule 5 entleert, muss für die nächste Behandlung eine neue Glaskarpule in die Karpulenhülse 4 eingelegt werden. Dazu wird die Karpulenhülse 4 über den Bajonettverschluss vom Gehäuse 2 der Spritze 1 getrennt, die Karpule ausgewechselt und die Karpulenhülse 4 anschließend wieder über den Bajonettverschluss mit dem Vorschubzylinder 8 verbunden.

Zusätzlich muss der Vorschubkolben 7 wieder in seine innere Endposition gebracht werden. Dazu muss die Spritze 1 in eine Ladestation eingelegt werden und über einen Hebelmechanismus der Vorschubkolben 7 zurückgeschoben werden. Hierzu betätigt die Ladestation den Tasterarm 23 und öffnet das Ventil der Steuerbohrung 20, damit eine Verbindung zwischen der ersten 16 und der zweiten Hydraulikkammer 19 hergestellt wird. Der Trennkolben 35 bewegt sich beim Rückschieben des Vorschubkolbens 7 zur inneren Endposition zurück und spannt das Gasvolumen im Druckraum 37 wieder auf den ursprünglichen Druck.

Zum nach mehrfachem Gebrauch eventuell notwendigen Nachfüllen oder auch zum Erstbefüllen der Spritze mit dem Federgas zeigt die Figur den Fülladapter 42. Über diesen kann der Druckraum 37 mit Stickstoff unter Druck befüllt werden. Das Rückschlagventil am O-Ring 41 verhindert dabei ein Ausströmen des Gases, wenn der Adapter abgeschraubt wird.

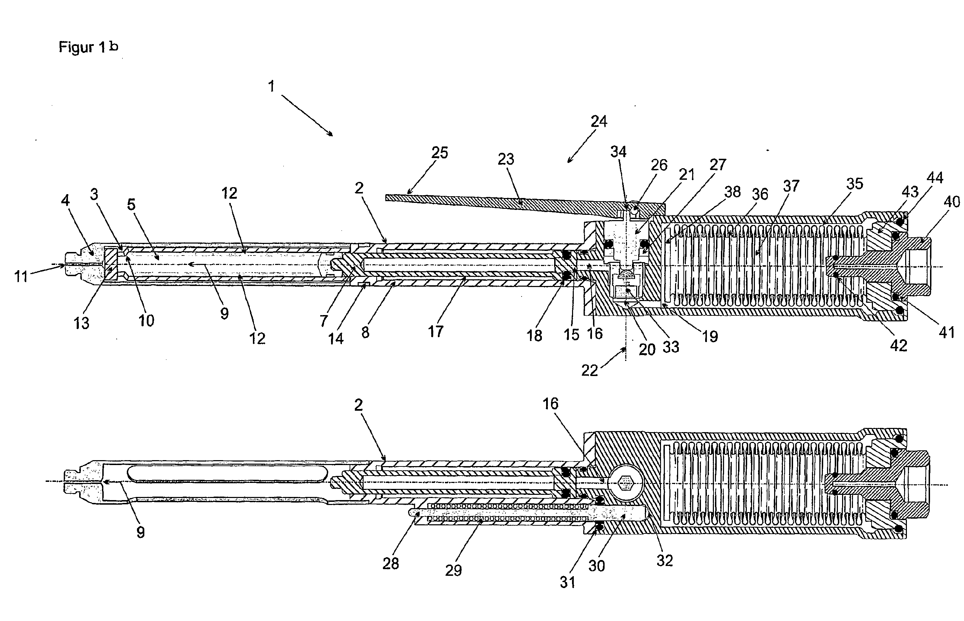

Die weitere Nummerierung bezieht sich auf das Ausführungsbeispiel der Figur 1b.

Die Anästhesiespritze 1 in der Figur 1b besteht im Wesentlichen aus einem Gehäuse 2, welches in einem Karpulenraum 3 einer Karpulenhülse 4 eine Karpule 5 aufnehmen und deren Inhalt mit einem hydraulisch-pneumatischen Antriebssystem 6 über einen Vorschubkolben 7 austreiben kann.

Der Vorschubkolben 7 ist in einem Vorschubzylinder 8 entlang einer Haupterstreckungsachse 9 der Anästhesiespritze 1 verschiebbar gelagert, wobei der Vorschubkolben 7 in der Figur eine innere Endlage einnimmt und bei einem Vorschub entlang der Achse 9 bis zu einer Anschlagschulter 10 bewegt werden kann, wo er eine äußere Endlage einnimmt. Beim Vorschub durchfährt der Vorschubkolben 7 den Karpulenraum 3, so dass Flüssigkeit aus einer eingelegten Karpule 5 durch eine Nadelöffnung 11 ausgespritzt werden kann. Zum Einsatz der Spritze 1 wird in die Nadelöffnung 11 eine Kanüle eingesetzt. Die Kanüle hat eine Spitze zum Einstechen ins Gewebe und eine zweite Spitze zum Einstechen in die Dichtmembran der eingelegten Karpule 5.

In der Nadelöffnung 11 ist eine Spezialaufnahme für die Kanüle vorgesehen, (nicht im Detail dargestellt), damit nicht versehentlich eine Kanüle in die Spritze 1 eingesetzt werden kann, welche bei den mitunter hohen Drücken mechanisch versagt.

Die Karpulenhülse 4 hat zwei Sichtfenster 12, durch welche die Vorschubposition des Vorschubkolbens 7 innerhalb der Karpulenhülse 4 einsehbar ist. Die Karpule 5 hat eine handelsübliche Kopfgestaltung 13, so dass der behandelnde Arzt nicht in der Wahl der Karpulen eingeschränkt wird, welche er bereits von der Arbeit mit herkömmlichen Spritzen kannte. Die Karpulenhülse 4 ist über einen Bajonettverschluss 14 mit dem Vorschubzylinder 8 fest verbunden.

Mit einer Druckplatte 15 ist der Vorschubkolben 7 einer ersten Hydraulikkammer 16 zugewandt, wobei eine Mantelfläche 17 des Vorschubkolbens 7 über ein Gleitdichtsystem 18 gegenüber der ersten Hydraulikkammer 16 abgedichtet ist.

Rückwärtig der ersten Hydraulikkammer 16 ist eine zweite Hydraulikkammer 19 vorgesehen und mit der ersten Hydraulikkammer 16 über eine Steuerbohrung 20 verbunden. Die Öffnung der Steuerbohrung 20 zwischen der ersten Hydraulikkammer 16 und der zweiten Hydraulikkammer 19 regelt ein Ventilschieber 21 über ein Rückschlagventil 33, wobei der Ventilschieber 21 entlang einer Bewegungsrichtung 22 verschiebbar gelagert ist und an einer Außenseite des Gehäuses 2 an einen Tasterarm 23 eines Steuertasters 24 stößt.

Dem vorderen Bereich der Spritze 1 zugewandt weist der Steuertaster 24 eine Tastfläche 25 zum Niederdrücken des Tasterarms 23 - und somit des Ventilschiebers 21 - auf Hierzu ist der Tasterarm 23 an einem Tasterlager 26 um einen Tasterbolzen drehbar gelagert. Der Tasterbolzen liegt senkrecht zur Haupterstreckungsachse 9 der Anästhesiespritze 1.

Der Ventilschieber 21 ragt mit einer Druckplatte 27 in die erste Hydraulikkammer hinein, während die Mantelfläche des Ventilschiebers 21 mit einem Gleitdichtsystem abgedichtet ist.

In ähnlicher Weise ist ein Indexkolben 28 im Gehäuse 2 entlang der Haupterstreckungsrichtung 9 der Spritze 1 gelagert. Der Indexkolben 28 ragt mit einem Fuß 30 in die erste Hydraulikkammer 16 hinein, während die Mantelfläche des Indexkolbens 28 mit einem Gleitdichtsystem 31 abgedichtet ist. Eine Druckfeder 29 presst den Indexkolben 28 zu einer inneren Endlage, bei welcher der Fuß 30 an einer inneren Begrenzungsfläche 32 zum Liegen kommt.

Ein Rückschlagventil 33 presst den Ventilschieber 21 unter Vorspannung im Ruhezustand in die dargestellte äußere Endlage, welche durch Anschlag der gegenüber dem zylindrischen Teil des Schiebers 21 vergrößert ausgeführten Druckplatte 27 an das Gehäuse 2 an einem Anschlag 34 definiert wird.

Die zweite Hydraulikkammer 19 ist rückwärtig von einem Gasspeicher 35 begrenzt, welcher entlang der Haupterstreckungsrichtung 9 der Spritze 1 deformierbar ausgeführt ist, und seinerseits einen in sich abgeschlossenen Druckraum 37 bildet und diesen damit begrenzt. Ein entlang der Haupterstreckungsrichtung 9 ziehharmonikaartig deformierbarer Metallbalg 36 bildet das wesentliche Element des Gasspeichers 35, wobei der Gasspeicher 35 in Richtung der zweiten Hydraulikkammer 19 durch eine Abdeckplatte 38 und rückwärtig mit einem Speicherdeckel 43 abgeschlossen wird, wobei ein frei von beweglichen Dichtelementen und vollständig vom Hydrauliköl getrennter Druckraum 37 gebildet wird. Der Gasspeicher 35 wird mit dem Speicherdeckel 43 über einen O-Ring 44 dicht an die zweite Hydraulikkammer 19 angeschlossen.

Der Druckraum 37 wird rückwärtig durch einen Verschlussstopfen 40 begrenzt, welcher über einen weiteren O-Ring 41 dicht an den Druckraum 37 angeschlossen ist und mit einem Rückschlagventil 42 versehen ist, über welches eine Gaspatrone oder ein Adapter zum Anschluss einer Druckgasleitung mit der Spritze 1 verbunden ist.

Im Betrieb wird der notwendige Hydraulikdruck zum Vorschub des Vorschubkolbens 7 vom Druckraum 37 erzeugt. Der Gasspeicher 35 trennt dabei das Gasvolumen (unter Druck über den Verschlussstopfer in den Druckraum 37 gefüllt) vom Hydrauliköl in der zweiten Hydraulikkammer 19. Über die Steuerbohrung 20 kann das unter Druck stehende Hydrauliköl zum Ventilschieber 21 gelangen. Der Ventilschieber wird über das Rückschlagventil 33 im Ruhezustand verschlossen. Das Rückschlagventil 33 ist in Sperrrichtung seriell zur zweiten Hydraulikkammer 19 angeordnet, um die notwendige Dichtkraft an der Steuerbohrung 20 zu erzeugen. Das Ventil ist ein Sitzventil mit proportionaler Charakteristik, somit kann abhängig vom Ventilhub der Volumenstrom und damit die Ausfuhrgeschwindigkeit des Vorschubkolbens 7 gesteuert werden.

Um den Vorschubkolben 7 auszufahren, muss der Tasterarm 23 bevorzugt an dessen Tastfläche 25 zum Gehäuse 2 der Spritze 1 hin betätigt werden. Dies verschiebt zwangsweise den Ventilschieber 21 entlang seiner Bewegungsrichtung 22 gegen die Druckfeder des Rückschlagventils 33 in das Gehäuse 2 der Spritze 1 hinein. Der freigegebene Strömungsquerschnitt kann nun vom Hydrauliköl von der zweiten Hydraulikkammer 19 zur ersten Hydraulikkammer 16 hin durchflossen werden. Somit wird der Kolbenboden 15 des Vorschubkolbens 7 mit Druck beaufschlagt, so dass der Vorschubkolben 7 ausfährt.

Liegt über das Gewebe, in welches die Spritze 1 eingestochen ist, eine Gegenkraft am Vorschubkolben 7 an, so steigt der Druck in beiden Hydraulikkammern, auf Grund der hydraulischen Verluste an der Steuerbohrung 20 jedoch insbesondere in der ersten Hydraulikkammer 16. Dieser Druck wirkt auf den Fuß 27 des Ventilschiebers 21 in Schließrichtung des Ventils. Der behandelnde Arzt spürt über den Taster 24 den veränderten Druck und muss entsprechend stärker auf den Taster 24 drücken, damit der Ventilschieber 21 in der geöffneten Position und damit das Ventil der Steuerbohrung 20 geöffnet bleibt.

Zusätzlich zu dieser Drucksensierung ist eine optische Druckanzeige über den Indexkolben 28 vorhanden, die den Zylinderdruck und damit die Kolbenkraft - welche als Maß für den Injektionsdruck angesehen werden kann - anzeigt. Dabei fungiert der Indexkolben 28 als kleiner einfachwirkender Hydraulikzylinder, an dessen Kolben drei Nuten zur Anzeige der Druckstärke eingebracht sind. Der Kolben 28 wird von der Feder 29 im Ruhezustand im Gehäuse 2 gehalten.

Bei ansteigendem Hydraulikdruck in der ersten Hydraulikkammer entsteht über den Fuß 30 des Indexkolbens 28 eine Kraft, welche der Federvorspannung entgegenwirkt und den Indexkolben 28 entsprechend der hydraulischen Druckverhältnisse in der ersten Hydraulikkammer 16 entlang der Bewegungsrichtung 9 aus dem Gehäuse 2 der Spritze 1 mehr oder weniger weit ausfährt. Über die Nuten am Kolben 28 kann dann der Druck abgelesen werden.

Ist der Vorschubkolben 7 komplett ausgefahren und somit die Glaskarpule 5 entleert, muss für die nächste Behandlung eine neue Glaskarpule in die Karpulenhülse 4 eingelegt werden. Dazu wird die Karpulenhülse 4 über den Bajonettverschluss vom Gehäuse 2 der Spritze 1 getrennt, die Karpule ausgewechselt und die Karpulenhülse 4 anschließend wieder über den Bajonettverschluss mit dem Vorschubzylinder 8 verbunden.

Zusätzlich muss der Vorschubkolben 7 wieder in seine innere Endposition gebracht werden. Dazu muss die Spritze 1 in eine Ladestation eingelegt werden und über einen Hebelmechanismus der Vorschubkolben 7 zurückgeschoben werden. Hierbei öffnet das Rückschlagventil 33, wobei eine Verbindung zwischen erster 16 und zweiter Hydraulikkammer 19 hergestellt wird. Der Gasspeicher 35 wird beim Rückschieben des Vorschubkolbens 7 komprimiert und spannt das Gasvolumen im Druckraum 37 wieder auf den ursprünglichen Druck.

Zum nach mehrfachem Gebrauch eventuell notwendigen Nachfüllen oder auch zum Erstbefüllen der Spritze mit dem Federgas wird ein Adapter an den Befüllstopfen 40 angeschlossen. Über diesen kann der Druckraum 37 mit Stickstoff unter Druck befüllt werden. Das Rückschlagventil am O-Ring 42 verhindert dabei ein Ausströmen des Gases, wenn der Adapter abgeschraubt wird.

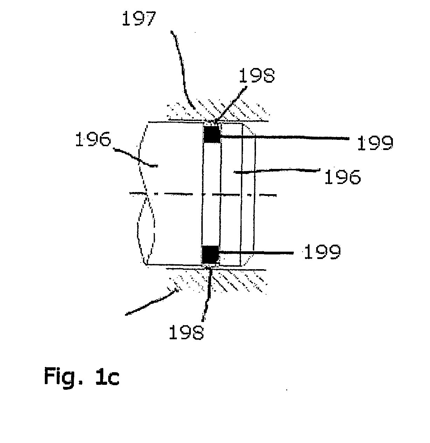

In der Figur 1c ist ein Gleitdichtsystem, ein Kolben 196 und ein Zylinder 197 dargestellt. Das Gleitdichtsystem umfasst den O-Ring 199 und einen Ring 198 aus PTFE, wobei der O-Ring 199 den Ring aus PTFE vorspannt. Der O-Ring 199 drückt aufgrund seiner Elastizität und der damit einhergehenden Rückstellkraft den Ring aus PTFE 198 gegen eine Wand des Zylinders 197. Somit kann der Kolben 196 in dem Zylinder 197 so geführt werden, dass ausbildende Räume links und rechts vom Gleitdichtsystem zueinander abgedichtet sind.

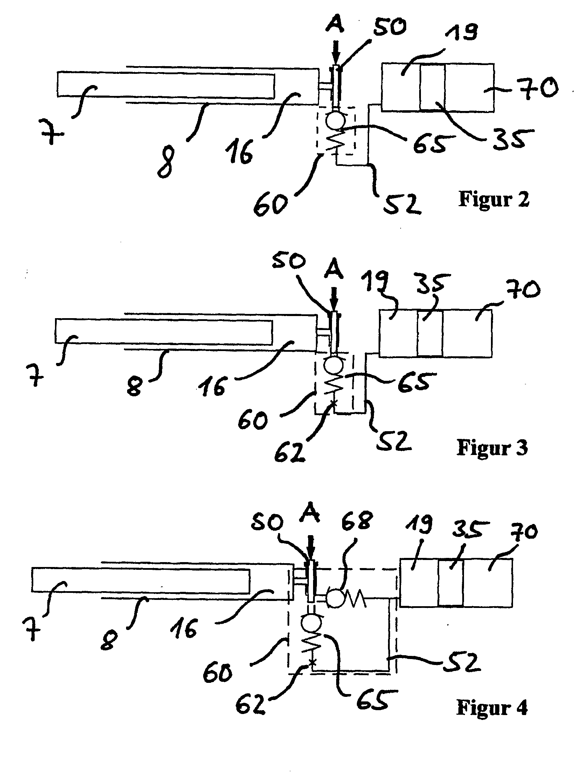

In den Figuren 2, 3 und 4 sind drei Möglichkeiten der Verwendung eines Rückschlagventils in einer Spritze beschrieben. Figur 2 stellt eine Variante mit einem Rückschlagventil 65 dar. Das Rückschlagventil 65 welches hier statt dem Vertikalschieber 21 eingesetzt wird, wird zur Steuerung der Spritze 1 eingesetzt, in dem der Dichtkörper des Ventils (hier dargestellt als eine Kugel) mit Hilfe eines Bestätigungsstifts oder entsprechend Pins 50 gegen die Federkraft in Richtung A verschoben und somit ein Steuerquerschnitt freigegeben wird.

Über den Steuerquerschnitt kann nun über die zweite Hydraulikkammer 19 Druck in der ersten Hydraulikkammer 16 aufgebaut werden. Dies wird über die Leitung 52 und dem Steuerelement 60, welches hier separat dargestellt ist, da dies ein handelsübliches Bauteil ist, realisiert. Der Gasdruck im Gasspeicher 70 kann über den Trennkolben 35 Druck in der zweite Hydraulikkammer aufbauen, welcher bei Betätigten des Steuerungselements über die zweite und erste Hydraulikkammer den Vorschubkolben 7 in den Karpulenraum 3 treibt.

In Figur 3 ist eine Variante mit einem Rückschlagventil 65 und einer Drossel 62, welches als Widerstandselement wirkt, dargestellt. Die Drossel 62 kann hierbei sowohl vor (wie hier dargestellt) als auch nach dem Rückschlagventil 65 angeordnet sein. Durch diese Ausgestaltungsform kann eine verbesserte Dosierbarkeit erreicht werden. Statt einer Drossel 62 kann auch eine Blende als Widerstandselement eingesetzt werden. Diese Elemente können auch so ausgestaltet sein, dass der Bediener das Widerstandselement verändert. Dies kann insbesondere durch eine Stellschraube, welche die Zuleitung 60 verengt, realisiert werden.

In Figur 4 ist eine Variante mit zwei Rückschlagventilen und einer Drossel 62 dargestellt. Im Vergleich zu Figur 3 ist das zweite Rückschlagventil 68, welches hier die Funktion des zweiten Steuerelements übernimmt, mit erstem und zweitem Hydraulikraum verbunden und realisiert somit eine Parallelschaltung von zweiten Steuerelement 68 und erstem Steuerelement 65 mit der in Reihe zum ersten Steuerelement 65 geschalteten Drossel 62.

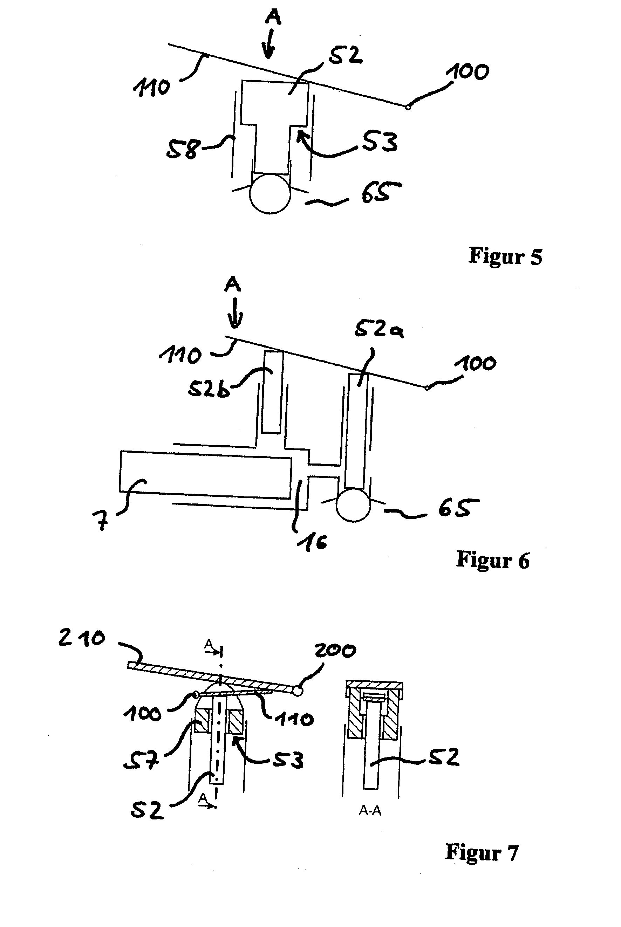

In den Figuren 5 bis 7 sind drei Möglichkeiten der Betätigung zum Öffnen des Rückschlagventils und zur Kraftrückführung für den Bediener dargestellt. In Figur 5 weist der Pin 52 eine umlaufende Ringfläche 53 auf, welche als Wirkfläche ausgestaltet ist, somit umfasst das Schaltelement einen Pin 52 mit Wirkfläche 53 und ein Rückschlagventil 65, wobei der Pin im Wesentlichen durch das Führungsmittel 58 geführt, ist. Das Schaltelement kann durch Betätigen in Richtung A über den Hebel 110, welcher sich um den Hebelpunkt oder entsprechend um die Hebelachse 100 dreht, bewegt oder entsprechend beschaltet werden.

In Figur 6 ist eine Wirkfläche räumlich getrennt vom ersten Pin 52a dargestellt. Das Schaltelement umfasst den ersten Pin 52a, welcher auf das Rückschlagventil 65 wirken kann, und den zweiten Pin 52b, welcher die Kraftrückkupplung über den ersten Hydraulikraum 16 erfährt, und den Hebel 110, welcher um den Hebelpunkt (Hebelachse) 100 geführt ist. Die benötigte Kraft auf den Betätigungspin ist in der selben Größenordnung wie die Rückkraft, soll jedoch vom Benutzer nicht gefühlt werden, da sie die haptische Wahrnehmung des wirksamen Drucks beeinträchtigt. Deshalb wird die Betätigung nahe an den Hebelachsen angeordnet.

Um die Rückkraft zu verstärken ist der zweite Pin 52b weiter als der erste Pin 52a vom Hebelpunkt (Hebelachse) 100 entfernt. Sowohl der Pin 52a als auch der Pin 52b werden über einen gemeinsamen Hebel(arm) 110 betätigt. Strömt bei Betätigung des Hebels 110 die Hydraulikflüssigkeit in die erste Hydraulikkammer 16 so der Druck, welcher in der Hydraulikkammer entsteht an den zweiten Pin 52b übertragen. Diese Kraft wird auf den Hebel 110 an den Bediener übermittelt.

In Figur 7 sind zwei getrennte, aber koaxiale Rückführflächen mit einem Hebelsystem dargestellt. Der rechte Teil der Figur stellt die Blickrichtung A-A dar. Der innere Pin 52 dient zum Öffnen des Rückschlagventils (nicht dargestellt), die Ringfläche 53 dient zur druckabhängigen Kraftrückführung. Die Betätigung des Pins 52 geschieht mittels eines ersten Hebels 110, 100 der an den zweiten Hebel 210, 220 gekoppelt ist. Somit werden unterschiedliche Hebelübersetzungen für zwei koaxial angeordnete Bauteile erzielt. Die Hebelachsen sind jeweils ortsfest im Gehäuse angebracht.

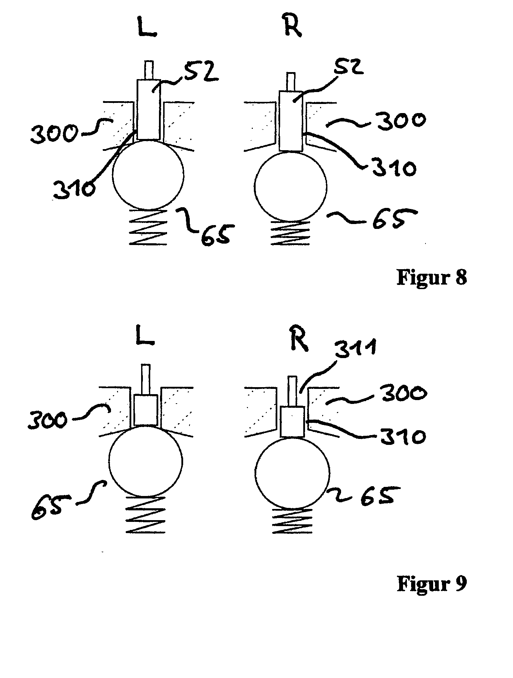

Die Figuren 8 und 9 zeigen Realisationen von Widerstandselementen. Auf der linken Seite L ist das unbeschaltete Rückschlagventil und auf der jeweils rechten Seite R das beschaltete Rückschlagventil dargestellt.

In Figur 8 ist ein Widerstandselement mit konstantem Widerstand dargestellt. Ein zwischen dem Betätigungspin 52 und dem Rückschlagventilgehäuse 300 befindlicher Spalt 310 dient zum Drosseln. Durch die Betätigung des Pins 52 wird die Länge und Höhe des Drosselspalts 310 nicht verändert. Die Drosselwirkung ist somit über den Hub konstant.

Figur 9 zeigt eine Drossel mit veränderlicher Lage über dem Hub, welches zu einer Veränderung des Widerstands führt. Links und rechts unterscheidet wieder das Schaltelement im betätigtem und nichtbetätigtem Zustand. Durch eine Änderung der Länge des Drosselspalts, welche durch den sich verengenden Querschnitt des Pins realisiert wird, kommt es zu einer hubabhängigen Drosselwirkung, welche zur Dosierung genutzt werden kann. Somit ist ein nicht konstanter Widerstand realisiert.

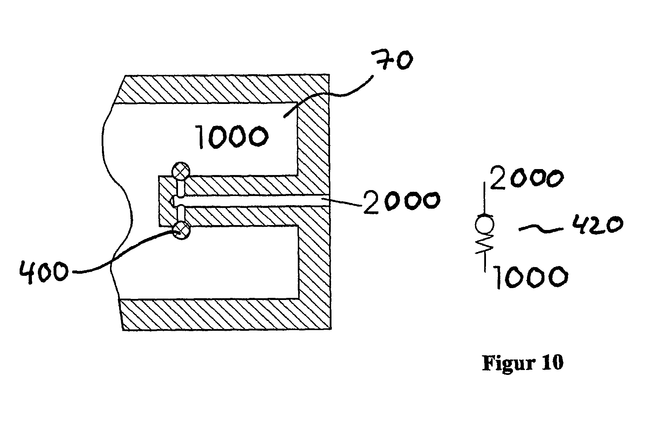

In Figur 10 ist der Gasspeicher 70, welcher über einen O-Ring 400 vom Befüllstutzen 2000 getrennt ist, dargestellt. Wobei hier der Gasspeicher 70 als ein erster Raum 1000 und der Befüllstutzen als ein zweiter Raum 2000 wirkt. Wird an dem Befüllstutzen beispielsweise eine Gaspatrone angeflanscht, kann der Druck im zweiten Raum 2000 den Druck im ersten Raum 1000 übersteigen, dabei dehnt sich das elastische Element 400, welches hier als O-Ring ausgestaltet ist, aus und der Druck im ersten Raum 1000 steigt an. Für die Wirkweise ist rechts das Ersatzschaltbild 420 als Rückschlagventil dargestellt.

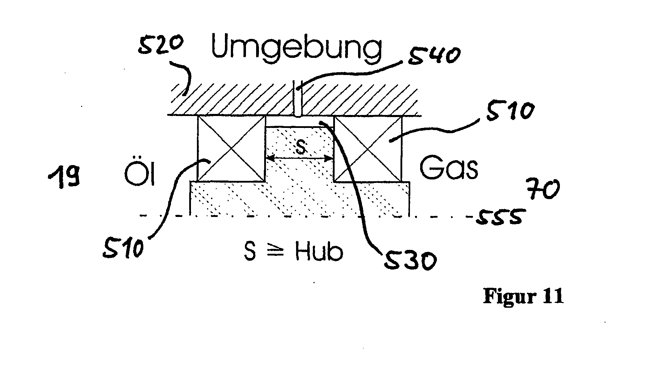

Um einen Übertritt eines Gases in eine Flüssigkeit wie beispielsweise Hydrauliköl zu verhindern, ist eine Vorrichtung vorgesehen, wie sie als Schnittzeichnung in Figur 11 dargestellt ist. Dabei ist der Übergang vom Gasraum 70 zur zweiten Hydraulikkammer 19, welche ein Öl aufweist, dargestellt. Die Dichtungen 510 dichten jeweils über den Kolben 35 die Systeme zueinander ab. Der Hub des Kolbens ist hier mit S bezeichnet. Zwischen dem Kolben und der Begrenzung 520 ist ein Hohlraum (Zwischenraum) 530 ausgebildet. Dieser Zwischenraum weist eine Öffnung 540 zur Umgebung auf. Über diese Öffnung kann ein Entgasen des Gases, welches durch die Dichtung in den Spalt gelangte, realisiert werden.

## Patentansprüche

1. Spritze (1), insbesondere Anästhesiespritze, mit einem längsverschieblichen Vorschubkolben (7), welcher über eine erste Hydraulikkammer (16) verschiebbar angeordnet ist, und einer zweiten Hydraulikkammer (19), wobei die erste und zweite Hydraulikkammer widerstandsregulierbar über ein erstes Steuerelement verbunden sind, wobei das erste Steuerelement (60) über ein Schaltelement betätigbar ist und das Schaltelement ein Betätigungsstift (50) ist, ***dadurch gekennzeichnet, dass*** der Betätigungsstift einem ersten Rückschlagventil (65) zugeordnet ist, sodass mittels des Betätigungsstifts das erste Rückschlagventil (65) betätigbar ist.

2. Spritze nach Anspruch 1, wobei zwischen erster und zweiter Hydraulikkammer ein Widerstandselement (62) in Reihe zum ersten Rückschlagventil geschaltet ist und somit insbesondere das erste Steuerelement ein erstes Rückschlagventil und ein Widerstandselement umfasst.

3. Spritze nach einem der vorhergehenden Ansprüche, wobei das Widerstandselement als Drossel oder Blende ausgestaltet ist.

4. Spritze nach einem der vorhergehenden Ansprüche, wobei zwischen erster und zweiter Hydraulikkammer ein zweites Steuerelement (68), insbesondere ein zweites Rückschlagventil parallel zum ersten Steuerelement geschaltet ist.

5. Spritze nach einem der vorhergehenden Ansprüche, wobei das Schaltelement einen Pin (50, 52) umfasst, welcher verschiebbar ist und bei Betätigung das erste Steuerelement öffnet, so dass die erste und zweite Hydraulikkammer miteinander leitend verbunden sind.

6. Spritze nach einem der vorhergehenden Ansprüche, wobei das Schaltelement eine Wirkfläche (53) umfasst, welche wenigstens einen Teil eines hydraulischen Drucks und somit einer wirkenden Kraft mittels des Stellelements überträgt.

7. Spritze nach einem der vorhergehenden Ansprüche, wobei die wirkende Kraft verstärkt wird.

8. Spritze nach einem der vorhergehenden Ansprüche, wobei die Verstärkung der wirkenden Kraft über einen weiteren Pin mit einer Kraftwirkung über einen Hebel (110) realisiert wird.

9. Spritze nach einem der vorhergehenden Ansprüche, wobei die Verstärkung der Kraft über ein Hebelsystem, von zwei miteinander verschalteten Hebeln erfolgt.

10. Spritze nach einem der vorhergehenden Ansprüche, wobei das Widerstandselement als Spalt (310) mit einer Spaltlänge um das Schaltelement insbesondere um den Pin ausgestaltet ist.

11. Spritze nach einem der vorhergehenden Ansprüche, wobei die Spaltlänge so ausgestaltet ist, dass sich die Spaltlänge beim Betätigen des Schaltelements verändert.

12. Spritze nach einem der vorherigen Ansprüche, wobei die Spritze einen Gasraum (1000) umfasst, welcher mit der zweiten Hydraulikkammer verbunden ist.

13. Spritze nach Anspruch 12, wobei der Gasraum über einen Befüllstutzen (2000) befüllbar ist und der Gasraum über einen O-Ring (400) gegenüber dem Befüllstutzen abgedichtet ist, wobei der O-Ring so ausgestaltet ist, dass die Funktion eines Rückschlagventils ausgeprägt wird.

14. Spritze nach einem der Ansprüche 12 oder 13, wobei eine Verbindung von Gasraum und zweiter Hydraulikkammer über einen beweglichen Trennkolben erfolgt.

15. Spritze nach einem der vorhergehenden Ansprüche, wobei der Trennkolben und zwei Dichtelemente, welche je den Gasraum und die zweite Hydraulikkammer abdichten, ein Kolbensystem ausbilden, wobei das Kolbensystem entlang eines Begrenzungselements führbar ist, wobei die Dichtungselemente, der Trennkolben und das Begrenzungssystem einen Hohlraum ausbilden, welcher eine Öffnung zur Umgebung realisiert.

16. Spritze nach einem der vorhergehenden Ansprüche, wobei die Öffnung in allen Trennkolbenpositionen austauschenden Kontakt zwischen Hohlraum und Umgebung ausbildet.

17. Spritze nach einem der Ansprüche 12 oder 13, wobei der Gasraum über einen dehnbaren, insbesondere vollständig geschlossenen, Gasspeicher, insbesondere ein Membranbalg, welcher als Federmembranspeicher wirkt, mit der zweiten Hydraulikkammer verbunden ist.

18. Spritze nach Anspruch 17, wobei der Gasspeicher über einen Befüllstutzen befüllbar ist.

19. Spritze nach einem der Ansprüche 17 oder 18, wobei der dehnbare Gasspeicher über eine verschließbare Entlüftungsöffnung verfügt und die Entlüftung des Gasraumes vor der Trennung des Gasspeichers von der zweiten Hydraulikkammer erfolgt.

## Claims

1. A syringe (1), in particular an anesthesia syringe having a feeding plunger (7) which is longitudinally displaceably so that it can be displaced over a first hydraulic compartment (16) and a second hydraulic compartment (19), wherein the first and second hydraulic compartments are connected by means of a first control element so that the resistance can be regulated, wherein the first control element (60) can be operated by means of a switch element, and the switch element is an actuating pin (50),
**characterized in that**
the actuating pin is assigned to a first check valve (65), so that the first check valve (65) can be actuated by means of the actuating pin.

2. The syringe according to Claim 1, wherein a resistance element (62) is connected in series with the first check valve between the first and second hydraulic compartments and thus in particular the first control element comprises a first check valve and a resistance element.

3. The syringe according to any one of the preceding claims, wherein the resistance element is designed as a throttle or an aperture.

4. The syringe according to any one of the preceding claims, wherein a second control element (68), in particular a second check valve is connected in parallel with the first control element between the first and second hydraulic compartment.

5. The syringe according to any one of the preceding claims, wherein the switch element comprises a pin (50, 52) which is displaceable and opens on actuation of the first control element, so that the first and second hydraulic compartments are connected to one another in a conducting manner.

6. The syringe according to any one of the preceding claims, wherein the switch element comprises an active area (53), which transmits at least a portion of the hydraulic pressure and thus an active force by means of the actuator element.

7. The syringe according to any one of the preceding claims, wherein the active force is potentiated.

8. The syringe according to any one of the preceding claims, wherein the potentiation of the active force is implemented by means of an additional pin having a force acting by way of a lever (110).

9. The syringe according to any one of the preceding claims, wherein the potentiation of the force is accomplished by means of a lever system of two interconnected levers.

10. The syringe according to any one of the preceding claims, wherein the resistance element is designed as a gap (310) having a gap length equal to the switch element, in particular around the pin.

11. The syringe according to any one of the preceding claims, wherein the gap length is designed so that the gap length is altered on actuation of the switch element.

12. The syringe according to any one of the preceding claims, wherein the syringe comprises a gas compartment (1000), which is connected to the second hydraulic compartment.

13. The syringe according to Claim 12, wherein the gas compartment can be filled by means of a filling connection (2000), and the gas compartment is sealed with respect to the filling connection by means of an O-ring (400), wherein the O-ring is designed so that the function of a check valve is embodied.

14. The syringe according to any one of Claims 12 or 13, wherein a connection of the gas compartment and the second hydraulic compartment is achieved by means of a movable dividing plunger.

15. The syringe according to any one of the preceding claims, wherein the dividing plunger and two sealing elements which seal the gas compartment and the second hydraulic compartment each form a plunger system wherein the plunger system can be guided along a bordering element, wherein the sealing elements, the dividing plungers and the bordering system form a hollow cavity, which implements an opening to the surroundings.

16. The syringe according to any one of the preceding claims, wherein the opening in all dividing plunger positions forms a replaceable contact between the hollow cavity and the surroundings.

17. The syringe according to any one of Claims 12 or 13, wherein the gas compartment is connected by means of an expandable gas storage mechanism, in particular one that is completely closed, in particular diaphragm bellows, acting as a spring diaphragm storage connected to the second hydraulic compartment.

18. The syringe according to Claim 17, wherein the gas reservoir can be filled by means of a filling connection.

19. The syringe according to any one of Claims 17 or 18, wherein the expandable gas reservoir has a closable vent opening and the venting of the gas space takes place before the separation of the gas reservoir from the second hydraulic compartment.

## Revendications

1. Seringue (1), en particulier seringue pour anesthésie, comportant un piston d'avancement coulissant longitudinalement (7) qui est disposé de manière à pouvoir être déplacé via une première chambre hydraulique (16) et une seconde chambre hydraulique (19), dans laquelle les première et seconde chambres hydrauliques ont une résistance réglable et sont reliées par un premier élément de commande, le premier élément de commande (60) étant actionnable à l'aide d'un élément de commutation et l'élément de commutation étant une tige d'actionnement (50), **caractérisée en ce que** la tige d'actionnement est associée à un premier clapet de retenue (65), de sorte que le premier clapet de retenue (65) est actionnable au moyen de la tige d'actionnement.

2. Seringue selon la revendication 1, dans laquelle, entre la première et la seconde chambre hydraulique, un élément de résistance (62) est branché en série avec le premier clapet de retenue et qu'en particulier le premier élément de commande comprend ainsi un premier clapet de retenue et un élément de résistance.

3. Seringue selon une des revendications précédentes, dans laquelle l'élément de résistance se présente sous forme d'un étrangleur ou d'un diaphragme.

4. Seringue selon une des revendications précédentes, dans laquelle, entre la première et la seconde chambre hydraulique, un second élément de commande (68), en particulier un second clapet de retenue, est branché parallèlement au premier élément de commande.

5. Seringue selon une des revendications précédentes, dans laquelle l'élément de commutation comprend une broche (50, 52) qui est mobile et ouvre, en cas d'actionnement, le premier élément de commande, de sorte que les première et seconde chambres hydrauliques restent reliées en conduction mutuelle.

6. Seringue selon une des revendications précédentes, dans laquelle l'élément de commutation comprend une surface active (53) qui transfère au moins une partie d'une pression hydraulique et donc d'une force active au moyen de l'élément de réglage.

7. Seringue selon une des revendications précédentes, dans laquelle la force active est amplifiée.

8. Seringue selon une des revendications précédentes, dans laquelle l'amplification de la force active se fait à l'aide d'une autre broche par effet de force exercée sur un levier (110).

9. Seringue selon une des revendications précédentes, dans laquelle l'amplification de la force se fait à l'aide d'un système de levage par deux leviers branchés ensemble.

10. Seringue selon une des revendications précédentes, dans laquelle l'élément de résistance est réalisé sous forme d'un intervalle (310) dont la longueur d'intervalle est conformée autour de l'élément de commutation, en particulier autour de la broche.

11. Seringue selon une des revendications précédentes, dans laquelle la longueur de l'intervalle est définie de manière à ce que la longueur de l'intervalle change en cas d'actionnement de l'élément de commutation.

12. Seringue selon une des revendications précédentes, dans laquelle la seringue comprend une chambre à gaz (1000) qui est reliée à la seconde chambre hydraulique.

13. Seringue selon la revendication 12, dans laquelle la chambre à gaz (1000) peut être remplie par une tubulure de remplissage (2000) et la chambre à gaz est isolée de la tubulure de remplissage par un joint torique (400), le joint torique étant conçu de manière à assurer la fonction d'un clapet de retenue.

14. Seringue selon une des revendications 12 ou 13, dans laquelle une liaison de la chambre à gaz et de la seconde chambre hydraulique est établie par un piston séparateur mobile.

15. Seringue selon une des revendications précédentes, dans laquelle le piston séparateur et deux éléments d'étanchéité qui isolent chacun la chambre à gaz et la seconde chambre hydraulique constituent un système de piston, le système de piston pouvant être guidé le long d'un élément de limitation, les éléments d'étanchéité, le piston séparateur et le système de limitation constituant une cavité qui constitue une ouverture vers l'environnement.

16. Seringue selon une des revendications précédentes, dans laquelle l'ouverture forme, dans toutes les positions du piston séparateur, un contact d'échange entre la cavité et l'environnement.

17. Seringue selon une des revendications 12 ou 13, dans laquelle la chambre à gaz est reliée via une réserve de gaz extensible, en particulier totalement fermée, en particulier un soufflet à membrane qui fait office de réserve de gaz à membrane à ressort, à la seconde chambre hydraulique.

18. Seringue selon la revendication 17, dans laquelle la réserve de gaz peut être remplie à l'aide d'une tubulure de remplissage.

19. Seringue selon une des revendications 17 ou 18, dans laquelle la réserve de gaz extensible dispose d'un orifice de purge refermable et la purge de la chambre à gaz a lieu avant la séparation de la chambre à gaz et de la seconde chambre hydraulique.
